# EUROPEAN PATENT APPLICATION

(11) **EP 4 414 367 A1**
(43) Date of publication of application: **14.08.2024**
(21) Application number: 22878696.8
(22) Date of filing: 08.08.2022
(51) Int. Cl.: C07D 493/04, A61K 31/37

(54) **NOVEL METHOD FOR SYNTHESIZING DECURSIN DERIVATIVE**

(30) Priority: 06.10.2021 KR 20210132240
(71) Applicant: PRG S&Tech Inc., Busan 46274 (KR)
(72) Inventor: KIM, Min Ju, Gijang-gun Busan 46081 (KR)
(74) Representative: Mewburn Ellis LLP
(86) International application number: PCT/KR2022/011763
(87) International publication number: WO 2023/058875

(57) **Abstract**

The present invention relates to a novel method for synthesizing a decursin derivative, enabling high-yield mass synthesis of a decursin derivative (PRG-A-04) represented by compound I through a reaction between decursinol and an intermediate compound in which a Boc protecting group is introduced to an OH group of 3-(3-methoxy-4-nitrophenyl)prop-2-en-1-ol in the presence of a platinum catalyst and xantphos.

## Description

### Technical Field

The present disclosure relates to a novel method of synthesizing a decursin derivative.

### Background Art

Amyotrophic lateral sclerosis (ALS) is a fatal neurodegenerative disease in which motor neurons are selectively lost by apoptosis in the spinal cord. About 10-20% of patients have a genetic pattern (familial ALS, fALS), while the rest are classified as sporadic ALS (sALS). Some genes have been identified to be on the ALS-associated gene locus in familial ALS, and SOD1 thereamong is the first gene identified in fALS. It is speculated that fALS-associated genes may play a role in the development of sALS, but the exact cause of sALS is currently unknown. In addition, ALS is classified, according to clinical symptoms, into typical ALS, ALS with dementia, and atypical ALS. In fact, mutations in SOD1 cause typical ALS, while C9orf72 is associated with ALS with dementia.

One of the important characteristics of ALS is that it is a progressive disease that the death of neurons is propagated through neural connections. In fact, Alzheimer's and Parkinson's diseases have similar phenotypes. In connection with the above feature, prion-like propagation has been proposed, in which a misfolded protein transforms a normal protein into an abnormal form. In fact, the mutant amyloid beta (Aβ) may transform normal Aβ into abnormal Aβ. Recently, it has been reported that mutated or misfolded SOD1 may also be secreted and propagated while the disease is in the progression. However, not much research has been conducted on SOD1 aggregation and misfolding inhibitors targeting treatments for ALS diseases.

Accordingly, in the Korea Patent Publication No. 10-2021-0052326, a decursin derivative compound, a novel compound, was proven to be effective in prevention, amelioration, or treatment of amyotrophic lateral sclerosis (ALS), but there have been limitations in mass production due to a low yield of decursin derivative compounds.

Therefore, research is required on a novel synthesis method that may increase the yield of the decursin derivative compound while enabling mass production.

### Disclosure of the Invention

### Technical Goals

An object of the present disclosure is to provide a method of synthesizing a novel decursin derivative, which increases a yield of decursin derivative compounds and enables mass production.

### Technical Solutions

In order to achieve the above object, the present disclosure provides a method of synthesizing a decursin derivative represented by Compound I, including synthesizing a decursin derivative represented by Compound I through a reaction between an intermediate compound (Compound 5), in which a Boc protecting group is introduced to an OH group of 3-(3-methoxy-4-nitrophenyl)prop-2-en-1-ol, and a decursinol (Compound 6).

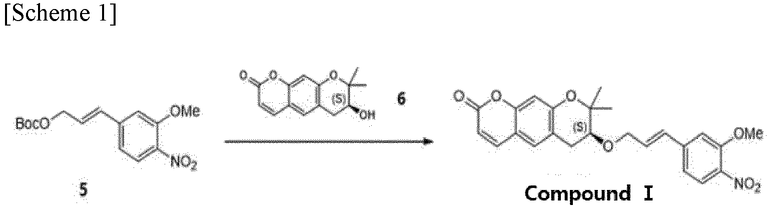

### Advantageous Effects

A novel method of synthesizing a decursin derivative compound according to the present disclosure has the advantages of being able to increase a yield of decursin derivative compounds obtained and enabling mass production.

### Brief Description of Drawings

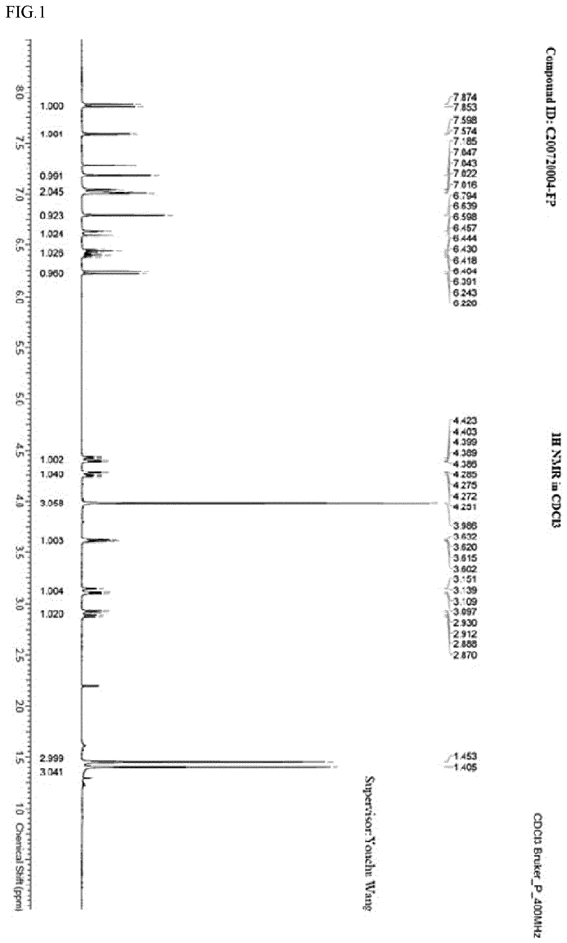

FIG. 1 shows a result of ¹H NMR for a decursin derivative (PRG-A-04) synthesized according to an example embodiment of the present disclosure;

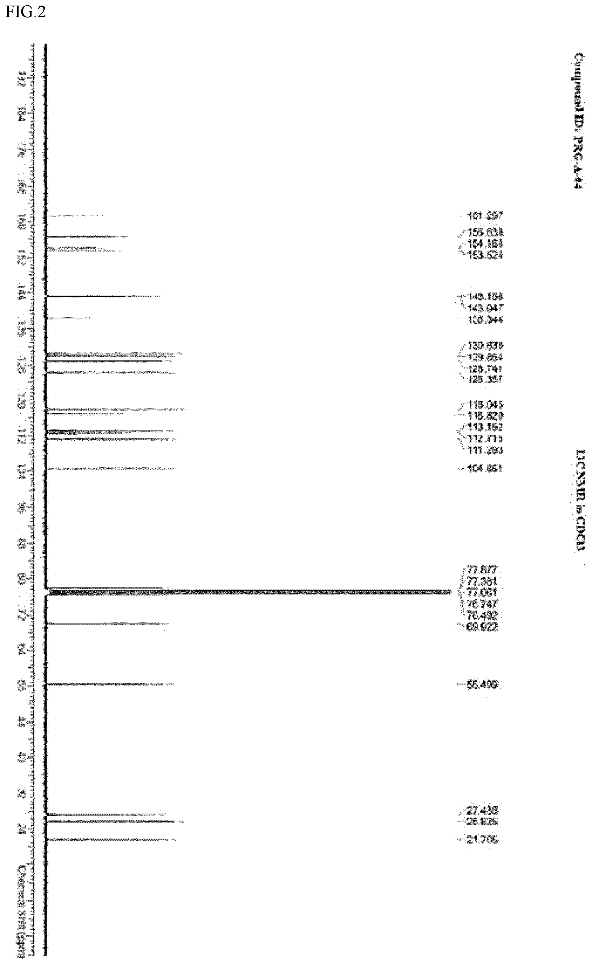

FIG. 2 shows a result of ¹³C NMR for a decursin derivative (PRG-A-04) synthesized according to an example embodiment of the present disclosure; and

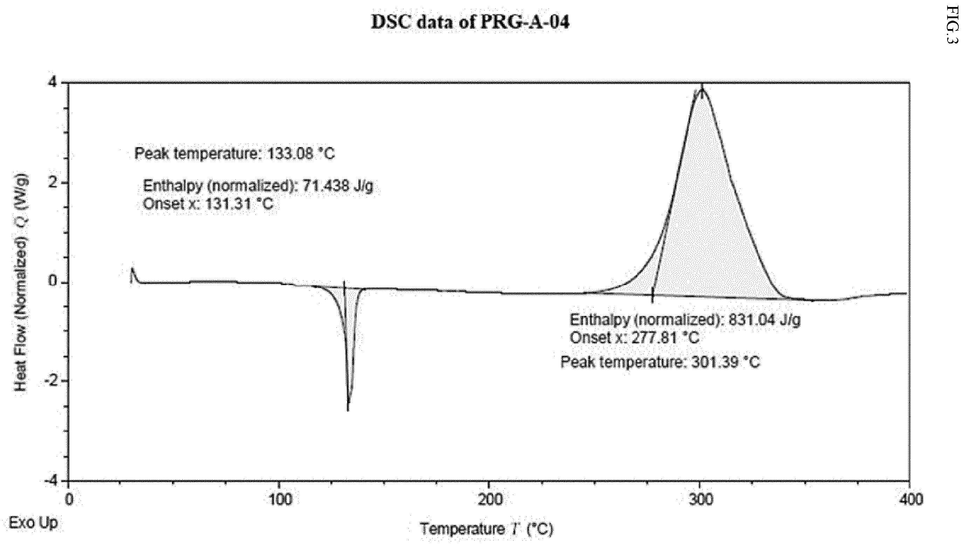

FIG. 3 shows a result of DSC for a decursin derivative (PRG-A-04) synthesized according to an example embodiment of the present disclosure.

### Best Mode for Carrying Out the Invention

Hereinafter, the present disclosure will be described in more detail.

The inventors of the present disclosure completed the present disclosure by developing a novel synthesis method that enables a high yield mass synthesis of (S,E)-7-((3-(3-methoxy-4-nitrophenyl)allyl)oxy)-8,8-dimethyl-7,8-dihydro-2H,6H-pyrano[3,2-g]chromen-2-one (PRG-A-04), a decursin derivative, represented by Compound I through a reaction between an intermediate compound, in which a Boc protecting group is introduced to an OH group of 3-(3-methoxy-4-nitrophenyl)prop-2-en-1-ol, and decursinol in the presence of a platinum catalyst and xantphos.

Accordingly, the present disclosure provides a synthesis method of a decursin derivative represented by Compound I, including synthesizing a decursin derivative represented by Compound I through a reaction between an intermediate compound (Compound 5), in which a Boc protecting group is introduced to an OH group of 3-(3-methoxy-4-nitrophenyl)prop-2-en-1-ol, and a decursinol (Compound 6).

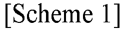

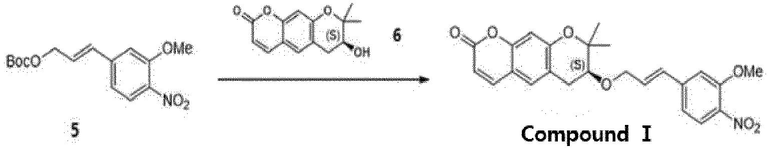

The Compound 5 may be synthesized by including: i) a first step of reacting 3-hydroxy-4-nitrobenzaldehyde with methyl iodide (CH₃I) to synthesize 3-methoxy-4-nitrobenzaldehyde; ii) a second step of reacting the 3-methoxy-4-nitrobenzaldehyde with diethoxyphosphoryl acetate to synthesize ethyl 3-(3-methoxy-4-nitrophenyl)-2-propenoate; iii) a third step of reacting the ethyl 3-(3-methoxy-4-nitrophenyl)-2-propenoate with diisobutylaluminium hydride (DIBAL-H) to synthesize 3-(3-methoxy-4-nitrophenyl)prop-2-en-1-ol; and iv) a fourth step of reacting the 3-(3-methoxy-4-nitrophenyl)prop-2-en-1-ol with di-tert-butyl decarbonate ((Boc)₂O) to synthesize Compound 5 with a Boc protecting group introduced.

The synthesizing of the decursin derivative may include synthesizing the decursin derivative represented by Compound I through a reaction between Compound 5 and Compound 6 in the presence of a platinum catalyst and xantphos.

More specifically, the synthesizing of the decursin derivative may include: i) adding a solution including Compound 6, Pd₂(dba)₃, and xantphos to a Compound 5 solution followed by stirring; ii) adding N-acetylcysteine to the stirred reaction solution followed by stirring; iii) filtering the stirred reaction solution and concentrating an organic solvent layer; iv) filtering the concentrated reaction solution to concentrate the collected solution and then adding acetone for complete dissolution; and v) adding isopropyl alcohol to the dissolved reaction solution and performing washing to obtain the decursin derivative represented by Compound I.

The Compound 5 and Compound 6 may be included in a weight ratio of 1:(0.1 to 1), and the platinum catalyst and xantphos may be each included in an amount of 3 to 10 parts by weight based on 100 parts by weight of the Compound 6. If the content of the Compound 5, Compound 6, platinum catalyst, and xantphos goes beyond the above range, it may cause issues such as economic infeasibility or difficulty in mass-production.

In the present disclosure, it is possible to further include recrystallizing the obtained decursin derivative.

The recrystallizing may include: i) adding acetone and isopropyl alcohol to the obtained decursin derivative followed by stirring and filtration; and ii) washing and drying the filtered reactant to obtain a decursin derivative.

To describe the present disclosure in more detail on the basis of an example embodiment of the present disclosure, the present disclosure provides a synthesis method of a decursin derivative represented by Compound I, including: a first step of reacting 3-hydroxy-4-nitrobenzaldehyde and methyl iodide (CH₃I) to synthesize 3-methoxy-4-nitrobenzaldehyde; a second step of reacting the 3-methoxy-4-nitrobenzaldehyde and diethoxyphosphoryl acetate to synthesize ethyl 3-(3-methoxy-4-nitrophenyl)-2-propenoate; a third step of reacting the ethyl 3-(3-methoxy-4-nitrophenyl)-2-propenoate and diisobutylaluminium hydride (DIBAL-H) to synthesize 3-(3-methoxy-4-nitrophenyl)prop-2-en-1-ol; a fourth step of reacting the ethyl 3-(3-methoxy-4-nitrophenyl)prop-2-en-1-ol and di-tert-butyl dicarbonate ((Boc)₂O) to synthesize Compound 5 with a Boc protecting group introduced; and a fifth step of reacting the Compound 5 with decursinol (Compound 6) to synthesize Compound I.

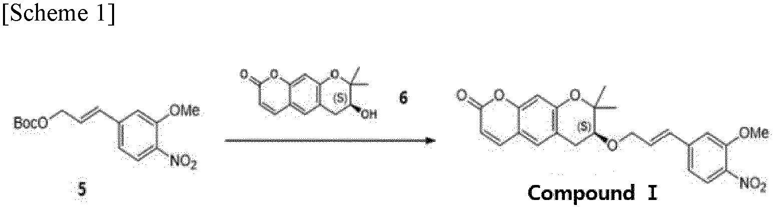

The method of synthesizing a decursin derivative (PRG-A-04) represented by Compound I according to the present disclosure is described in more detail in the following Scheme 2:

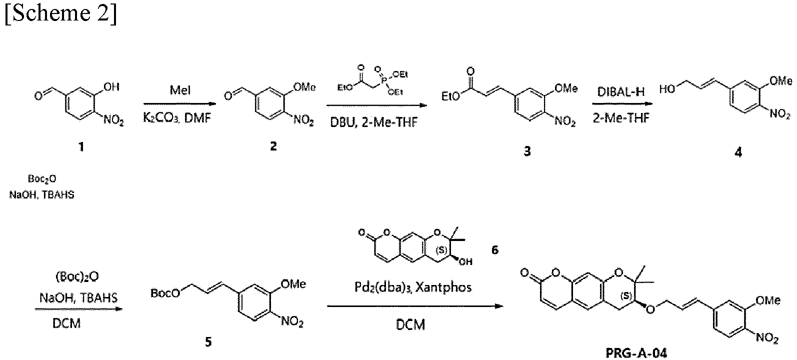

The first step may include: putting 3-hydroxy-4-nitrobenzaldehyde (Compound 1), K₂CO₃, and DMF into a reactor and then adjusting an internal temperature of the reaction solution to 20-30°C; while maintaining a temperature of the reaction solution at 20-40°C, adding MeI and DMF to the reaction solution followed by stirring for 16-18 hours while maintaining a temperature of the reaction solution at 20-30°C; lowering the temperature of the reaction solution to -5-10°C and then adding purified water while maintaining a temperature of the reaction solution at -5-10°C, followed by stirring at -5 to 10°C for 2-6 hours; and filtering solids resulting from the reaction solution and drying the filtrate under reduced pressure to obtain 3-methoxy-4-nitrobenzaldehyde (Compound 2).

The second step may include: putting 2-diethoxyphosphoryl acetate (DBU) and 2-Me-THF into a reactor followed by stirring; adding a solution mixed with 3-methoxy-4-nitrobenzaldehyde (Compound 2) and 2-Me THF to the reaction solution, then stirring the mixture at 20-30°C for 8-12 hours, adding purified water, and stirring the mixture at 20-30°C for 30-60 minutes; and distilling, under reduced pressure, an organic solvent layer obtained by treating an acetic acid solution and an NaHCO₃ solution to the organic solvent layer of the stirred reaction solution to obtain 3-(3-methoxy-4-nitrophenyl)prop-2-en-1-ol (Compound 3).

The third step may include: putting 3-(3-methoxy-4-nitrophenyl)prop-2-en-1-ol (Compound 3) and 2-Me-THF into the reactor followed by stirring; adding DIBAL-H (toluene solution, 96.5 kg, 112.2 moles, 2.5 eq) to the reaction solution followed by stirring; adding a seignette salt solution to the reaction solution followed by stirring; and distilling, under reduced pressure, an organic solvent layer obtained by treating the seignette salt solution, an acetic acid solution, and an NaHCO₃ solution to the organic solvent layer of the stirred reaction solution and then adding 2-Me THF to obtain 3-(3-methoxy-4-nitrophenyl)prop-2-en-1-ol (Compound 4).

The fourth step may include: putting a 3-(3-methoxy-4-nitrophenyl)prop-2-en-1-ol (Compound 4) solution and dichloromethane (DCM) into a reactor followed by distillation under reduced pressure; adding DCM to the reaction solution distilled under reduced pressure; adding tetra-butyl-ammonium-hydrogen-sulfate (TBAHS), di-tert-butyl dicarbonate ((Boc)₂O), and DCM to the reaction solution followed by stirring; adding aqueous NaOH solution to the reaction solution followed by stirring; and obtaining a Compound 5 solution which is an organic solvent layer obtained after treating the organic solvent layer of the stirred reaction solution with an acetic acid solution and an NaHCO₃ solution.

The fifth step may include: adding a solution including Compound 6, Pd₂(dba)₃, and xantphos into the Compound 5 solution followed by stirring; adding N-acetylcysteine to the stirred reaction solution followed by stirring; filtering the stirred reaction solution and concentrating the organic solvent layer; filtering the concentrated reaction solution, concentrating the collected solution, and adding acetone for complete dissolution; and adding isopropyl alcohol to the dissolved reaction solution and performing washing to obtain a decursin derivative (PRG-A-04) represented by Compound I.

In addition, the decursin derivative (PRG-A-04) represented by Compound I synthesized according to the present disclosure, i.e., (S,E)-7-((3-(3-methoxy-4-nitrophenyl)allyl)oxy)-8,8-dimethyl-7,8-dihydro-2H,6H-pyrano[3,2-g]chromen-2-one, is an inhibitor of SOD1 aggregation and misfolding according to Republic of Korea Patent Publication No. 10-2021-0052326, which may be useful as a treatment for ALS diseases by showing protective effects on muscle weakness and movement disorders in ALS model mice. Modes for Carrying Out the Invention

Hereinafter, the present disclosure will be described in more detail through example embodiments. These example embodiments are merely intended to describe the present disclosure more specifically, and it will be apparent to those of ordinary skill in the art to which the present disclosure pertains that the scope of the present disclosure is not limited by the example embodiments according to the gist of the present disclosure.

### <Example 1> Synthesis method of PRG-A-04

A synthesis method of PRG-A-04 according to the present disclosure was synthesized according to the following Scheme 2.

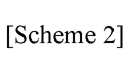

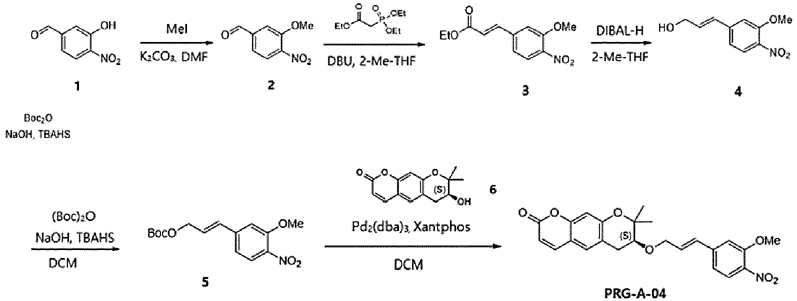

A more detailed synthesis method of PRG-A-04 is as follows.

### 1. Synthesis of Compound 2

Compound 2 was synthesized according to the following Scheme 3.

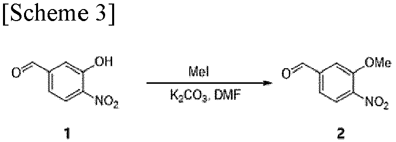

Compound 1 (17.9 kg, 107.1 mol, 1 eq), K₂CO₃ (20.5 kg, 148.3 moles, 1.4 eq), and DMF (80 kg) were put into a reactor, and an internal temperature of the reaction solution was adjusted to 25°C. While maintaining a temperature of the reaction solution at 30°C, MeI (33.8 kg, 238.1 moles, 2.2 eq) and DMF (11 kg) were added to the reaction solution, and stirring was performed while maintaining a temperature of the reaction solution at 25°C. After lowering the temperature of the reaction solution to 5°C, purified water (180 kg) was added while maintaining a temperature of the reaction solution at 5°C and stirred for 4 hours. (Stirring temperature: 5°C)

The solids resulting from the reaction solution thus prepared were filtered (an internal temperature of a filter: 5°C), and the filtered solids were washed with purified water (72 kg x 2 times). The filtered solids were placed in a dryer and dried under reduced pressure for 20 hours to obtain Compound 2 (yield: 17.53 kg (90.4%), purity: 99.9% (HPLC), content: 99.2% (w/w), residual DMF<0.0442% (w/w), residual MeI: 176 ppm) (an internal temperature of the dryer: 50°C).

### 2. Synthesis of Compound 3

Compound 3 was synthesized according to the following Scheme 4.

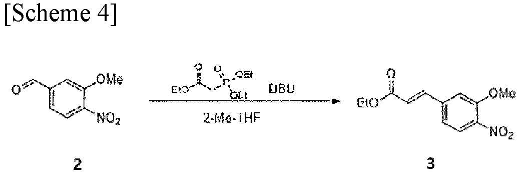

2-diethoxyphosphoryl acetate (DBU, 20.5 kg, 91.4 moles, 1.1 eq.) and 2-Me-THF (130 kg) were put into a reactor, and the reaction solution was adjusted to 0°C. While maintaining a temperature of the solution at 0°C, DBU (15.5 kg, 101.8 moles, 1.2 eq.) was added and stirred at 0°C for 2 hours. A solution mixed with Compound 2 (15 kg, 83.0 mol, 1.0 eq) and 2-Me THF (225 kg) was added to the reaction solution, and a temperature of the reaction solution was maintained at 0°C. After stirring the reaction solution at 25°C for 10 hours, purified water (82 kg) was added, followed by stirring at 25°C for 45 minutes. After stopping the stirring and waiting at 25°C for 45 minutes until the solution layer is separated, the water layer was discarded by separating the water layer and the organic solvent layer, and 10% aq. acetic acid solution (a solution mixed with purified water (156 kg) and glacial acetic acid (16.0 kg), 83 kg) was added to the organic solvent layer, followed by stirring at 25°C for 45 minutes. After stopping the stirring and waiting at 25°C for 45 minutes until the solution layer is separated, the water layer was discarded by separating the water layer and the organic solvent layer, and 9.6% aq. NaHCO₃ solution (a solution mixed with purified water (176 kg, 11.7 X) and NaHCO₃ (18.1kg), 84kg) was added to the organic solvent layer, followed by stirring at 25°C for 45 minutes. After stopping the stirring and waiting at 25°C for 45 minutes until the solution layer separate is separated, the water layer was discarded by separating the water layer and the organic solvent layer, and 9.6% aq. NaHCO₃ solution was added and stirred to separate the solution layer, wherein the process was performed repeatedly. The organic solvent layer thus obtained was distilled under pressure (a reactor temperature: maintained below 50°C), and distillation was halted when the volume of the solution decreased by approximately 60 L. N-heptane (130 kg) was added while maintaining at 25°C for 2 hours, the reaction solution was stirred at 25°C for 6 hours, and the filtered solid was washed with n-heptane (17.0 kg). The filtered solids were placed in a dryer and dried under reduced pressure for 20 hours (an internal temperature of the dryer: 50°C) to obtain Compound 3 (yield: 18.35kg (88 %), purity: 99.7% (HPLC), content: 99.1% (w/w), residual moisture: 0.1% (KF)).

### 3. Synthesis of Compound 4

Compound 4 was synthesized according to the following Scheme 5.

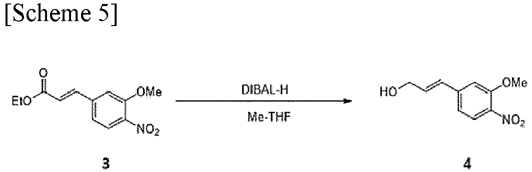

First, after checking that the residual moisture (KF measurement) of 2-Me-THF to be used as a solvent is less than 0.05%, the moisture that might possibly remain in a reactor was completely dried out. Compound 3 (11.1 kg, 44.2 moles, 1.0 eq) and 2-Me-THF (205 kg) were put into the reactor, and a reaction solution was adjusted to -5-5°C. 1M DIBAL (toluene solution, 96.5 kg, 112.2 moles, 2.5 eq) was added slowly for more than 2 hours while maintaining a temperature of the solution at 0°C and stirred for 3 hours while maintaining a temperature at 0°C. After adjusting a temperature of the reaction solution to 25°C, 38.7% aq. seignette salt solution (a solution mixed with purified water (126 kg) and potassium sodium tartrate quadro hydrate (83.4 kg), 113 kg] was added and stirred for 45 minutes while maintaining at 25°C. After stopping the stirring and waiting at 25°C for 3 hours to ensure proper separation of the solution layer, the water layer was discarded, 38.7% aq. seignette salt solution (58 kg) was added to the organic solvent layer, and stirring was performed for 0.5-1 hour while maintaining at 25°C. After stopping the stirring and waiting at 25°C for 2.5 hours to ensure proper separation of the solution layer, the water layer was discarded, and 10% aq. acetic acid solution (60 kg) was added to the organic solvent layer, followed by string while maintaining at 25°C. After stopping the stirring and waiting at 25°C for 45 minutes to ensure proper separation of the solution layers, the water layer was discarded, and 9.6% aq. NaHCO₃ (60 kg) was added to the organic solvent layer, followed by string for 45 minutes while maintaining at 25°C. After stopping the stirring and waiting at 25°C for 45 minutes until the solution layer is separated, the water layer was discarded, and 9.6% aq. NaHCO₃ solution was added and stirred to separate the solution layer, wherein the process was performed repeatedly. The resulting organic solvent layer was distilled under reduced pressure (a temperature of the reactor: maintained below 50°C), and when the solution volume decreased by approximately 27 L, distillation was stopped. 2-Me THF (54 kg), a solvent, was added, the organic solvent layer was distilled under reduced pressure (a temperature of the reactor: maintained below 50°C), the distillation was stopped when the volume of solution was reduced by approximately 27 L, and distillation was performed under reduced pressure by adding solvent 2-Me THF, wherein the process was repeated. After completion of distillation under reduced pressure, 2-Me THF (120 kg) was added, and the residual moisture in the solution was checked to be less than 1.0% (yield: 150 kg of Compound 4 solution (in 2-Me-THF), purity: 84.5% (HPLC), content: 4.1% (w/w), residual moisture: 0.4% (KF)).

### 4. Synthesis of Compound 5

Compound 5 was synthesized according to the following Scheme 6.

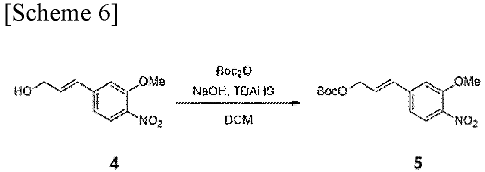

Compound 4 solution (in 2-Me-THF, 6.0 kg, 4.1 w/w%) and dichloromethane (DCM, 46 kg) were put into the reactor and distilled under reduced pressure (a temperature of the reactor: maintained below 50°C). When the volume of the solution was reduced to approximately 9 L, distillation was stopped. DCM (46 kg) was added and distilled under reduced pressure (a temperature of the reactor: maintained below 50°C). When the volume of the solution was reduced to approximately 9 L, distillation was halted, and the process was repeated. DCM (80 kg) was added, and stirring was performed at 25°C for 1 hour. Tetra-butyl-ammonium-hydrogen-sulfate (TBAHS, 0.8 kg, 2.4 moles, 0.1 eq), di-tert-butyl decarbonate ((Boc)₂O, 12.5kg, 57moles, 2.0eq), and DCM (14kg) were added to the reaction solution, followed by stirring at 25°C. 5.4% aq. NaOH solution (a solution mixed with purified water (108 kg) and NaOH (6.10 kg, 152.5 moles, 5.3 eq), 80.0 kg) was slowly added for 1 hour while maintaining a temperature of the solution at 25°C, and stirring was performed for 9 hours at the same temperature. After stopping the stirring and waiting at 25°C for 45 minutes to ensure separation of the solution layers, the water layer was discarded, and 10% aqueous acetic acid solution (a solution mixed with purified water (61.4 kg) and acetic acid glacial (12.0 kg), 36.2 kg) was added to the organic solvent layer, followed by stirring at 25°C for 45 minutes. After stopping the stirring and waiting at 25°C for 45 minutes to ensure separation of the solution layer, the water layer was discarded, and the 10% aqueous acetic acid solution was treated, whein the process was performed repeatedly. 9.6% aqueous NaHCO₃ solution (a solution mixed with purified water (162 kg) and NaHCO₃ (16.9 kg), 36.2kg) was added to the organic solvent layer, and stirring was performed at 25°C for 45 minutes. After stopping the stirring and waiting at 25°C for 45 minutes to ensure separation the solution layer, the water layer was discarded, and after repeating the process, the organic solvent layer (Compound 5 solution (in DCM): 100.45 kg, purity: 88.9% (HPLC), content: 9.3% (w/w)) was obtained.

### 5. Synthesis of PRG-A-04

PRG-A-04 was synthesized according to the following Scheme 7.

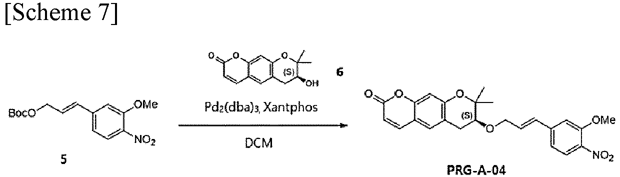

First, the dried reactor was filled with nitrogen, and Compound 6 (3.6 kg, 14.61 moles, 1.0 eq) was added while continuously charging nitrogen into the reactor. After nitrogen/vacuum was exchanged alternately three times in an attempt to remove the residual air inside the reactor, and at the end, the inside of the reactor was filled with nitrogen. Pd₂(dba)₃ (tris(dibenzylideneacetone)dipalladium(0), 202 g, 0.22 moles, 0.015 eq) and xantphos (255 g, 0.44 moles, 0.03 eq.) were put into the reactor, and after alternatively exchanging nitrogen/vacuum three times, the inside of the reactor was finally filled with nitrogen.

After dissolving Compound 5 (8.1 kg, 26.3 moles, 1.8 eq.) in DCM (18 L.), gas was removed. The solution was added to a solution consisting of Compound 6, Pd₂(dba)₃, and xantphos for four hours while slowly maintaining an internal temperature of the solution at 42°C, and after stirring at 42°C for 2 hours, the solution temperature was adjusted to 25°C. N-acetylcysteine (0.9 kg) was added and stirred at 25°C for 18 hours. The reaction solution was filtered using a strainer with a diatomite pad (1.0 kg) placed, and the filtered wet cake was washed with DCM (10 L). The filtered solution was washed twice with water (18L x 2), the water layer was discarded, and the organic solvent layer was concentrated to 10 L at a temperature below 50°C.

Using a strainer with a silica gel pad (10 kg) placed, the reaction solution was filtered, and the silica gel pad was washed using DCM to collect the reactants remaining on the silica gel pad. The collected solution was then concentrated to 5.4 L, acetone (-14.4 L) was added, the solution was concentrated to 5.4 L, acetone (-7.2 L) was added, the solution was concentrated to 5.4 L, acetone (-7.2 L) was added, and the reaction solution was stirred at 35°C until all solids were completely dissolved.

Isopropyl alcohol (IPA. 18 L) was stirred for 5 hours while maintaining a temperature of the solution at 35°C, the reaction solution was adjusted to 0°C for 3 hours, stirred at 0°C for 18 hours, and filtered, and then the filtered solid was washed using IPA (7.2 L).

In order to recrystallize the obtained solid, solid, acetone (7.2 L), and IPA (14.4 L) were put into the cleanly prepared reactor, stirring was performed at 50°C for 8 hours, the temperature of the solution was adjusted to 25°C over 3 hours, and then filtration was performed after stirring at 25°C for 8 hours. The filtered solid was washed using IPA (3.6 L) and dried at 52°C for 18 hours to obtain light yellow powder of PRG-A-04 (yield: 4.03 kg (63%), purity: 98.8 % (HPLC), chiral purity: 99.6 % (HPLC), residual moisture: 0.02 % (KF), loss on drying: 0.23 %, melting point (DSC): onset 131.31°C (peaktemp. 133.08°C), 277.81°C (peak temp. 301.39°C), residual palladium (Pd): 17 ppm, residual Xantphos: 80 ppm, residual Pd₂(dba)₃: 104 ppm).
¹H NMR (CDCl₃, 400MHz): δ 7.85(d,J=8.4Hz,1H), 7.57(d,J=9.5Hz,1H), 7.16(s,1H), 7.05-6.97(m,2H), 6.78(s,1H), 6.60(app.dt,J=15.9,1.7Hz,1H), 6.40(app.dt,J=15.9,5.5Hz,1H), 6.22(d,J=9.4Hz,1H), 4.39(ddd,J=13.6,5.4,1.7Hz,1H), 4.24(ddd,J=13.4,5.5,1.6Hz,1H), 3.97(s,3H), 3.59(dd,J=7.1,4.9Hz,1H), 3.11(dd,J=16.7,4.9Hz,1H), 2.87(dd,J=16.7,7.2Hz,1H), 1.43(s,3H), 1.38(s,3H)
¹³C NMR (CDCl₃, 151MHz): δ 161.4, 156.8, 154.4, 153.7, 143.2, 143.1, 138.5, 130.7, 130.0, 128.8, 126.5, 118.2, 116.9, 113.4, 112.9, 111.4, 104.9, 78.0, 76.7, 70.1, 56.6, 27.6, 26.0, 21.8;
IR (film): 2921, 2851, 1722, 1625, 1603, 1585, 1561, 1511, 1132 cm⁻¹;
HRMS: m/z calcd for C₂₄H₂₄NO₇⁺[M+H]⁺: 438.1547, found: 438.1550

Having described in detail a specific part of the contents of the present disclosure above, it is clear for those skilled in the art that this specific description is merely a preferred example embodiment, and the scope of the present disclosure is not limited thereby. Therefore, the substantial scope of the present disclosure is defined by the appended claims and their equivalents.

## Claims

1. A method of synthesizing a decursin derivative represented by Compound I, comprising synthesizing a decursin derivative represented by Compound I through a reaction between an intermediate compound (Compound 5), in which a Boc protecting group is introduced to an OH group of 3-(3-methoxy-4-nitrophenyl)prop-2-en-1-ol and a decursinol (Compound 6):

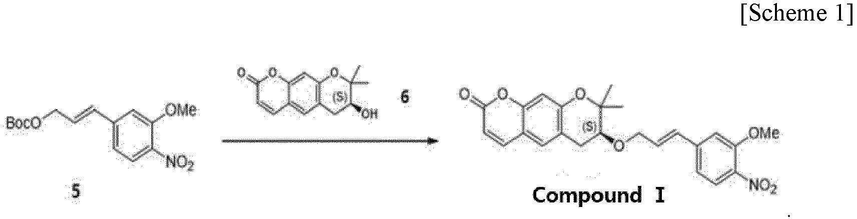

2. The synthesis method of claim 1, wherein the Compound 5 is synthesized by comprising: i) a first step of reacting 3-hydroxy-4-nitrobenzaldehyde with methyl iodide (CH₃I) to synthesize 3-methoxy-4-nitrobenzaldehyde; ii) a second step of reacting the 3-methoxy-4-nitrobenzaldehyde with diethoxyphosphoryl acetate to synthesize ethyl 3-(3-methoxy-4-nitrophenyl)-2-propenoate; iii) a third step of reacting the ethyl 3-(3-methoxy-4-nitrophenyl)-2-propenoate with diisobutylaluminium hydride (DIBAL-H) to synthesize 3-(3-methoxy-4-nitrophenyl)prop-2-en-1-ol; and iv) a fourth step of reacting the 3-(3-methoxy-4-nitrophenyl)prop-2-en-1-ol with di-tert-butyl decarbonate ((Boc)₂O) to synthesize Compound 5 with a Boc protecting group introduced.

3. The synthesis method of claim 1, wherein the synthesizing of the decursin derivative comprises synthesizing the decursin derivative represented by Compound I through a reaction between Compound 5 and Compound 6 in the presence of a platinum catalyst and xantphos.

4. The synthesis method of claim 1, wherein the synthesizing of the decursin derivative comprises: i) adding a solution comprising Compound 6, Pd₂(dba)₃, and xantphos to a Compound 5 solution followed by stirring; ii) adding N-acetylcysteine to the stirred reaction solution followed by stirring; iii) filtering the stirred reaction solution and concentrating an organic solvent layer; iv) filtering the concentrated reaction solution to concentrate the collected solution and then adding acetone for complete dissolution; and v) adding isopropyl alcohol to the dissolved reaction solution and performing washing to obtain the decursin derivative represented by Compound I.

5. The synthesis method of claim 4, wherein the Compound 5 and the Compound 6 are included in a weight ratio of 1:(0.1 to 1).

6. The synthesis method of claim 4, wherein the platinum catalyst and xantphos are each included in an amount of 3 to 10 parts by weight based on 100 parts by weight of the Compound 6.

7. The synthesis method of claim 4, further comprising recrystallizing the obtained decursin derivative.

8. The synthesis method of claim 7, wherein the recrystallizing comprises: i) adding acetone and isopropyl alcohol to the obtained decursin derivative followed by stirring and filtration; and ii) washing and drying the filtered reactant to obtain a decursin derivative.
